# EUROPÄISCHE PATENTSCHRIFT

(11) **EP 2 585 002 B1**
(45) Veröffentlichungstag und Bekanntmachung des Hinweises auf die Patenterteilung: **03.04.2019**
(21) Anmeldenummer: 11730606.8
(22) Anmeldetag: 22.06.2011
(51) Int. Cl.: A61F 2/46, A61F 2/00, A61F 2/30

(54) **EINSETZINSTRUMENT ZUM EINSETZEN VON PFANNENEINSÄTZEN IN HÜFTPFANNEN FÜR DIE HÜFTENDOPROTHETIK**
INSERTION INSTRUMENT FOR INSERTING SOCKET INSETS INTO HIP SOCKETS FOR HIP ENDOPROSTHESES
INSTRUMENT D'INSERTION POUR LA MISE EN PLACE D'INSERTS ACÉTABULAIRES POUR PROTHÈSES DE HANCHE

(30) Priorität: 24.02.2011 DE 102011004689; 25.06.2010 DE 102010030540
(43) Veröffentlichungstag der Anmeldung: 01.05.2013
(73) Patentinhaber: CeramTec GmbH, 73207 Plochingen (DE)
(72) Erfinder: PREUSS, Roman, 73230 Kirchheim unter Teck (DE); WOLF, Heike, 72622Nürtingen (DE); WEISS, Tobias, 73061 Ebersbach (DE); KILCHENMANN, Thomas, CH-8810 Horgen (CH); GRIESMAYR, Götz, 73650 Winterbach (DE); MUHR-SCHENK, Manuela, 70736 Fellbach (DE)
(86) Internationale Anmeldenummer: PCT/EP2011/060455
(87) Internationale Veröffentlichungsnummer: WO 2011/161166

(56) Entgegenhaltungen:
- WO-A1-2008/106598
- WO-A2-2011/095575
- DE-A1- 10 148 022
- DE-A1- 19 628 193
- DE-A1-102009 054 633
- DE-U1- 29 922 792
- FR-A1- 2 701 206
- US-B1- 6 468 281

## Beschreibung

Die Erfindung betrifft ein Einsetzinstrument zum instrumentierten Einsetzen eines Pfanneneinsatzes mit einer kugelförmigen Kalotte in eine Hüftpfanne einer Hüftgelenkprothese mit einem Impaktierinstrument mit einem Stiel, an dessen einem Ende sich ein Haltewerkzeug für den Pfanneneinsatz befindet.

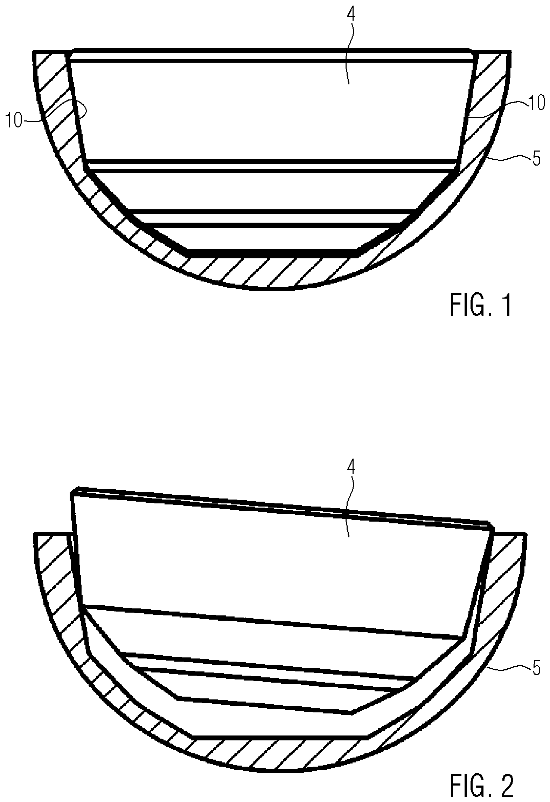

Im Markt existiert eine Vielfalt von Prothesensystemen zum Ersatz des natürlichen Hüftgelenks im Falle schmerzhafter traumatischer, arthrotischer oder anderer Veränderungen. Dabei werden in der Regel sogenannte modulare Systeme verwendet, bei denen in eine zumeist aus einer Metallegierung bestehende Hüftpfanne ein Pfanneneinsatz eingesetzt wird, welcher einen Teil des künstlichen Gleitlagers bildet und welcher aus einer Metallegierung, einem Keramikwerkstoff, einem Kunststoff oder einem Verbund aus den vorgenannten Materialien bestehen kann. Häufig erfolgt die Kopplung zwischen Pfanneneinsatz und Hüftpfanne über eine sogenannte konische Klemmung, bei der ein konisch geformter Teil der äußeren Geometrie des Pfanneneinsatzes mit einem passend konisch geformten Teil der inneren Geometrie der Hüftpfanne eine reibschlüssige Verbindung bildet, siehe Figur 1.

Eines der Probleme, welche intra-operativ auftreten können, ist das verkippte Einsetzen des Pfanneneinsatzes in die Hüftpfanne. Dann kann statt der beschriebenen konischen Klemmung ein Verklemmen des Pfanneneinsatzes zwischen drei Kontaktpunkten innerhalb des Klemmkonus der Hüftpfanne auftreten. Je nach der Höhe, der beim Verklemmen aufgebrachten Kraft, treten infolge der punktuellen Verklemmung so hohe Reibkräfte auf, dass die Position des Pfanneneinsatzes intra-operativ nicht mehr korrigiert werden kann, siehe Figur 2.

Die Folgen für die Funktion der Hüftgelenksprothese bei verkippt eingesetztem Pfanneneinsatz hängen wesentlich vom Material des Pfanneneinsatzes ab und reichen von erhöhtem Verschleiß über Korrosion bis hin zur vollständigen Zerstörung des Pfanneneinsatzes. So kann ein verkippt eingesetzter Pfanneneinsatz die Ursache für eine spätere aufwendige, schmerzhafte und teure Revisionsoperation sein.

Um das verkippte Einsetzen von Pfanneneinsätzen zu vermeiden, sind im Markt eine Reihe von Einsetzinstrumenten verfügbar. Deren Funktion basiert im wesentlichen auf den folgenden drei Schritten:
1. Greifen des Pfanneneinsatzes am oberen äußeren Rand.
2. Ausrichten des Instrumentes mit Pfanneneinsatz an der Hüftpfanne, so dass die Symmetrieachsen von Hüftpfanne und Pfanneneinsatz parallel verlaufen.
3. Ruckartiges, schnelles Schieben des Pfanneneinsatzes in die Hüftpfanne, dabei Aufhebung des Haltegriffes und Erzeugen der Klemmverbindung.

In EP 1 076 537 B1 und DE-U- 299 22 792 werden Einsetzinstrumente beschrieben, bei denen der Pfanneneinsatz von drei Halteklauen am oberen Rand gegriffen wird. Weiterhin bieten diese Einsetzinstrumente ein kurzes Griffstück, an dem der Operateur das Einsetzinstrument greifen und samt Pfanneinsatz ins OP-Feld einführen kann. Dort erfolgt das Aufsetzen auf die Hüftpfanne und schlußendlich das Fügen des Pfanneneinsatzes. Die Nachteile der Lösungen - geringe palpatorische Rückmeldung für den Operateur, benötigter Bauraum des Instruments erschwert Einsetzen und Verpackung - werden durch ein Einsetzinstrument gemäß DE 10 2009 054633 gelöst.

Als nachteilig wird von verschiedenen Operateuren die direkte manuelle Handhabung empfunden, bei der die Hand direkt ins OP-Feld geführt und evtl. das Wundgewebe berührt wird. Dies kann aus Sicht der Operateure zur Erhöhung des Infektionsrisikos führen. Weiterhin wird dadurch die Sicht auf das OP-Feld kurzzeitig stark eingeschränkt. Aus den genannten Gründen bevorzugen manche Operateure die Handhabung von Prothesenkomponenten über Instrumente mittels langem Stiel und endseitigem Griff.

Die DE10148022 zeigt ein Instrument zum Setzen einer künstlichen Beckenpfanne in ein Acetabulum mit einer Hantel und einem am Kopfende der Hantel angeordneten Aufnahme für die Beckenpfanne, weche mittels eines Kugelgelenks frei verschwenkbar ist.

In WO 2008/106598 wird ein Einsetzinstrument beschrieben, bei dem eine Art elastischer Deckel auf den Pfanneneinsatz gedrückt wird. Der Deckelrand greift den Pfanneneinsatz dabei auf dem gesamten Umfang. Durch eine Öffnung im Deckel inkl. radialer Schlitzung wird ein Impaktierinstrument bzw. die Kombination aus Pfanneneinsatz und Deckel auf ein Impaktierinstrument aufgesteckt. Mittels des Impaktierinstrumentes wird der Pfanneinsatz ins OP-Feld eingebracht und gefügt.

Nachteile der beschriebenen Lösung sind:
- Die Kopplung zwischen Deckel und Imapktierinstrument ist aufgrund der radialen Schlitzung der Deckelöffnung zwar geringfügig nachgiebig, erlaubt aber keine größere Verkippung des Pfanneneinsatzes mit Deckel relativ zur Achse des Imapktierinstrumentes. Das heißt, der Pfanneneinsatz ist beim Einführen ins OP-Feld im Wesentlichen senkrecht zur Achse des Instrumentenstieles ausgerichtet. Die Ausrichtung von Pfanneneinsatz und Hüftpfanne erfolgt also nicht selbsttätig, sondern muß durch den Operateur aktiv vorgenommen werden.
- Der Deckelrand umschließt den gesamten Umfang des Pfanneneinsatzes und muß zur korrekten Ausrichtung des Pfanneneinsatzes, relativ zur Hüftpfanne, auch über dem kompletten Umfang auf der Pfannenstirnfläche aufliegen. Sollte dies aufgrund überstehender Gewebereste, z.B. Osteophyten, nicht möglich sein, ist keine korrekte Ausrichtung des Pfanneneinsatzes vor dem Impaktieren möglich. Infolgedessen steigt das Risiko des verkippten Einsetzens bzw. ist das Instrument nicht anwendbar.
- Aufgrund der kompletten Umschließung des Pfanneneinsatzes durch den Deckel wird möglicherweise die Sicht auf die Stirnfläche der Hüftpfanne beim Einsetzen des Pfanneneinsatzes behindert, so dass die korrekte Ausrichtung des Pfanneneinsatzes, relativ zur Hüftpfanne, nicht visuell kontrolliert werden kann.

Der Erfindung liegt die Aufgabe zugrunde, ein Einsetzinstrument nach dem Oberbegriff des Anspruchs 1 so weiterzubilden, dass beim Einsetzen ein Verkippen des Pfanneneinsatzes ausgeschlossen ist und das Einsetzinstrument ein stielförmiges Impaktierinstrument umfasst.

Dadurch, dass das eine Ende des stielförmigen Impaktierinstruments kugelförmig ausgebildet und ein Teil des Haltewerkzeugs ist und das Haltewerkzeug weiterhin einen als separates Bauteil ausgebildeten Impaktierkopf zur gelenkigen Aufnahme des kugelförmigen Endes des Impaktierinstruments und eine als separates Bauteil ausgebildete Einsetzhilfe zum Halten des Pfanneneinsatzes auf der Außengeometrie des Impaktierkopfs umfasst und der Impaktierkopf mit dem kugelförmigen Ende ein Kugelgelenk bildend verbindbar ist, ist beim Einsetzen ein Verkippen des Pfanneneinsatzes ausgeschlossen. Das Einsetzinstrument umfasst ein stielförmiges Impaktierinstrument.

Bevorzugt ist die Außengeometrie des Impaktierkopfs an die Geometrie der Kalotte des Pfanneneinsatzes angepasst, wodurch der Impuls beim Einsetzen gleichmäßig auf die Kalotte im Pfanneneinsatz übertragen wird.

In einer Ausführungsform verfügt der Impaktierkopf über eine Kugelkalotte mit nachgiebigem Kalottenrand, in die das kugelförmige Ende des Impaktierinstruments bei Montage einschnappt, wodurch das lösbare Befestigen des Impaktierkopfs vereinfacht ist.

Erfindungsgemäss besteht die Einsetzhilfe aus einem Federring mit radial abstehenden Halteklauen, wobei die Halteklauen aufgrund der Nachgiebigkeit des Federrings radial verschiebbar sind und am äußeren Ende der Halteklauen axial abstehende Haken angeordnet sind, die im montierten Zustand mit ihrer Haltefläche an der Außenfläche des Pfanneneinsatzes anliegen und zugleich die Halteklauen mit ihrer Auflagefläche auf dem Rand des Pfanneneinsatzes aufliegen. Hierdurch lässt sich das Verkippen vermeiden.

Bevorzugt ist der Kalottenrand koaxial von einer umlaufenden Nut umgeben. Diese Nut kann den Federring der Einsetzhilfe im montierten Zustand aufnehmen.

In bevorzugter Ausführungsform liegen der Federring und die Halteklauen im montierten Zustand in einer Ebene oberhalb der Kalotte des Pfanneneinsatzes, was die Handhabung erleichtert.

Bevorzugt ist oberhalb des kugelförmigen Endes des Impaktierinstruments eine Auskragung zur Bewegungsbegrenzung des Kugelgelenks angeordnet. Diese Auskragung ist am Stiel umlaufend ausgebildet.

Damit die Einsetzhilfe nicht vom Impaktierinstrument abrutscht, ist bevorzugt der radiale Durchmesser des Impaktierkopfes größer als der Durchmesser des Federringes der Einsetzhilfe.

Ein erfindungsgemäßes Einsetzinstrument zum instrumentierten Einsetzen eines Pfanneneinsatzes mit einer kugelförmigen Kalotte in eine Hüftpfanne einer Hüftgelenprothese besteht aus
- einem Impaktierinstrument mit einem Stiel dessen eines Ende kugelförmig ausgebildet ist,
- einem als separates Bauteil ausgebildeten Impaktierkopf zur gelenkigen Aufnahme des kugelförmigen Endes des Impaktierinstruments und
- einer als separates Bauteil ausgebildeten Einsetzhilfe zur lösbaren Befestigung des Pfanneneinsatzes.

Das erfindungsgemässe Einsetzinstrument kann beispielsweise in einem Verfahren zum Einsetzen eines Pfanneneinsatzes mit einer kugelförmigen Kalotte in eine Hüftpfanne einer Hüftgelenprothese eingesetzt werden, welches die Schritte umfasst,
- dass zuerst ein zum Gleitpaarungsdurchmesser des Pfanneneinsatzes passender Impaktierkopf in die Kalotte des Pfanneinsatzes gesteckt wird,
- dass anschließend eine zum Außendurchmesser des Pfanneneinsatzes passende Einsetzhilfe über den Pfanneneinsatz mit eingelegtem Impaktierkopf gesetzt wird und die Halteklauen der Einsetzhilfe über den Rand des Pfanneneinsatzes gezogen werden, so dass der Pfanneneinsatz sicher von den Halteklauen gehalten wird,
- dass anschließend der im Pfanneneinsatz gelagerte Impaktierkopf auf das kugelförmige Ende des Impaktierinstruments gesteckt und die Schnappverbindung Kugel-Kalotte gefügt wird.

Das weitere Vorgehen beim Einsetzen umfasst die Schritte,
- dass anschließend mit dem Impaktierinstrument mit befestigten Pfanneneinsatz der Pfanneneinsatz in die Hüftpfanne hineinbewegt wird, bis die Halteklauen der Einsetzhilfe an ihrer Unterseite die Stirnfläche der Hüftpfanne berühren und der Pfanneneinsatz achsparallel zur Hüftpfanne ausgerichtet ist,
- dass anschließend ein Impuls auf das andere freie Ende des Impaktierinstruments gegeben wird und dadurch der Impaktierkopf schlagartig den Pfanneneinsatz in Richtung Hüftpfanne beschleunigt und dadurch der Pfanneneinsatz aus den Halteklauen und in weiterhin ausgerichteter Lage in die Hüftpfanne geschoben wird, bis eine Verankerung der beiden Komponenten eintritt.

Nachfolgend wird die Erfindung anhand von Figuren weiter erläutert. Gleiche Bezugszeichen bezeichnen auch den gleichen Gegenstand.
Figur 1 zeigt eine Hüftpfanne 5 in die ein Pfanneneinsatz 4 über eine konische Klemmung 10 korrekt eingesetzt ist.
Figur 2 zeigt eine Hüftpfanne 5 in die ein Pfanneneinsatz 4 verkippt eingesetzt ist. Die vorgesehene konische Klemmung 10 kommt nicht zum Tragen. Dies ist zu vermeiden.

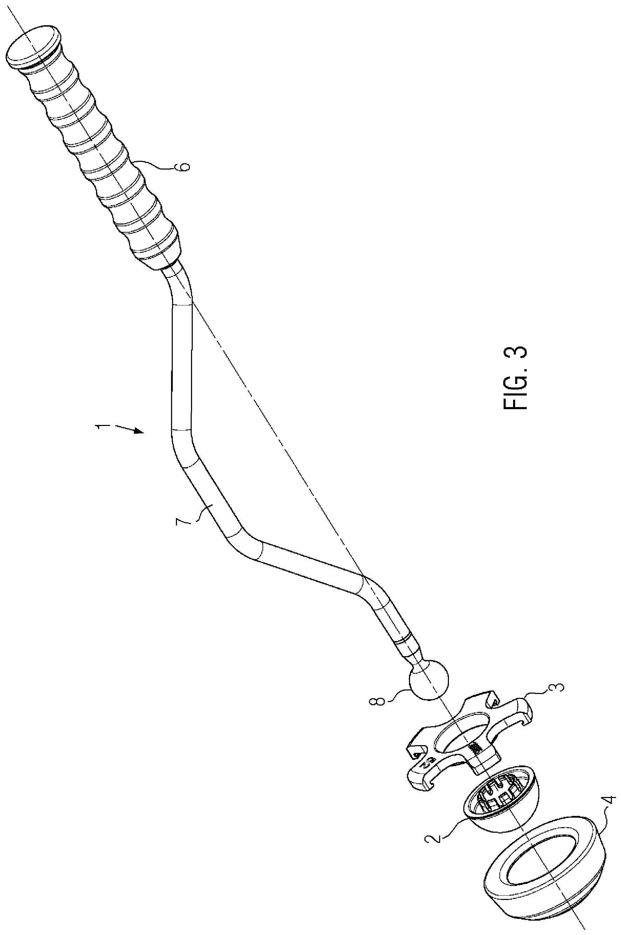

Figur 3 zeigt das Einsetzinstrument bestehend aus Impaktierinstrument 1 mit Haltegriff 6, Stiel 7 und einem kugelförmigen Ende 8, das ein Teil eines Kugelgelenks ist und aufsteckbarem Impaktierkopf 2 mit einer Kalotte als das zweite Teil des Kugelgelenks zur Aufnahme des kugelförmigen Endes 8 und einer Einsetzhilfe 3.

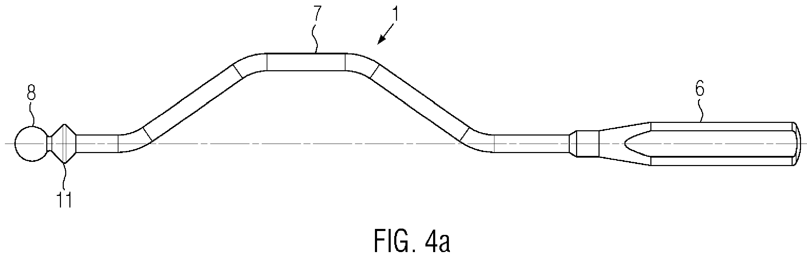

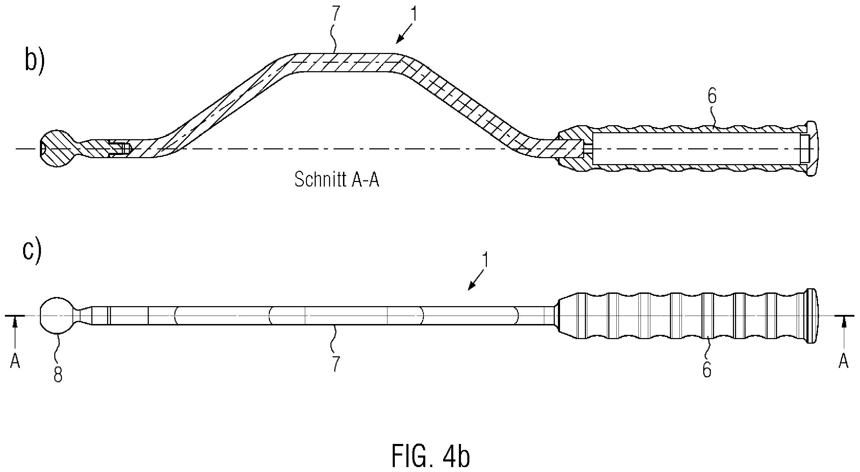

Figur 4 a zeigt ein Impaktierinstrument 1 mit einem Stiel 7, der an einem Ende in einen Haltegriff 6 übergeht und entgegengesetzt ein kugelförmiges Ende 8 als Teil eines Kugelgelenks aufweist. In der abgebildeten Ausführungsform ist der Stiel 7 gekröpft. Am kugelförmiges Ende 8 ist eine Auskragung 11 als Verkippungsanschlag angeordnet. Figur 4 b zeigt ein alternatives Impaktierinstrument 1 ohne Verkippungsanschlag und Figur 4 c den Schnitt A-A nach Figur 4 b.
Figur 5 a zeigt in Draufsicht von oben einen erfindungsgemäßen Impaktierkopf 2 und Figur 5 b den Schnitt A-A gemäß Figur 5 a.

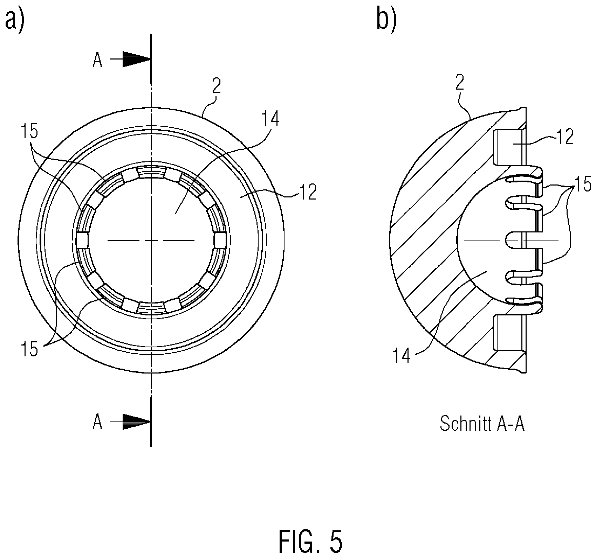

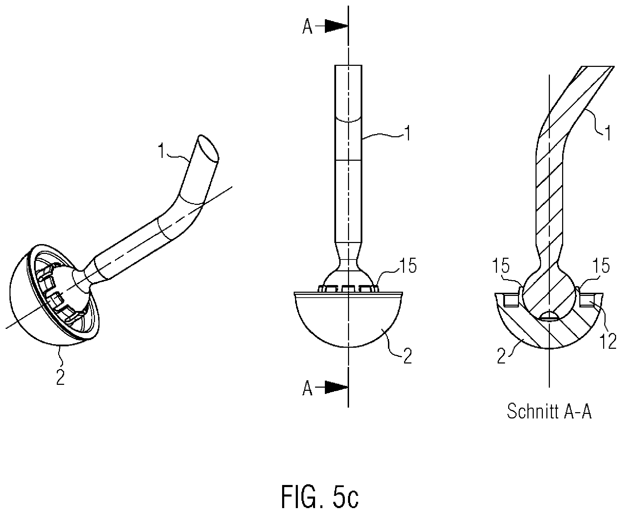

Figur 5 c zeigt das Impaktierinstrument 1 mit aufgesetztem Impaktierkopf 2 in Ansichten und einem Schnitt. Der aufsteckbare Impaktierkopf 2 ist mit einer umlaufenden Nut 12 zur Aufnahme des Federringes 13 der Einsetzhilfe 3 versehen. Die Kugelkalotte 14 weist einen nachgiebigen, über den Kugeläquator hinaus erhöhten Kalottenrand 15 auf. Das Impaktierinstrument 1 ist mit dem Impaktierkopf 2 mit kugelförmigem Ende 8 als Kugelgelenk montiert.

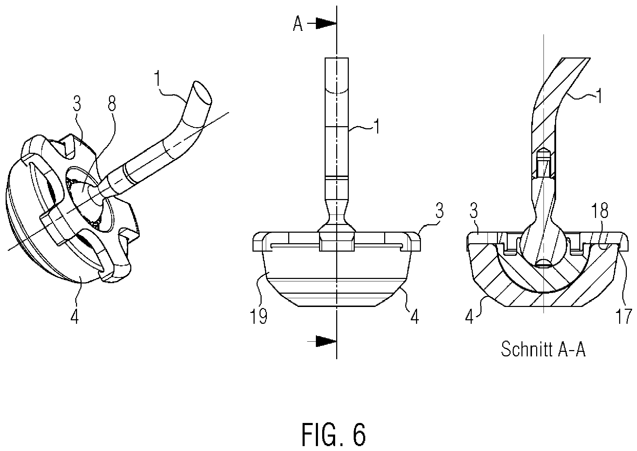

Figur 6 zeigt das Impaktierinstrument 1 montiert mit Einsetzhilfe 3, Impaktierkopf 2 und Pfanneneinsatz 4 - fertig für intra-operatives Einsetzen des Pfanneneinsatzes 4 in die Hüftpfanne 5.

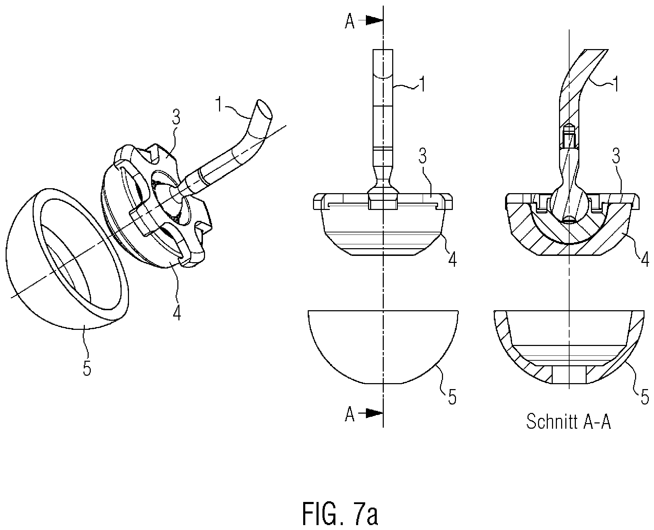

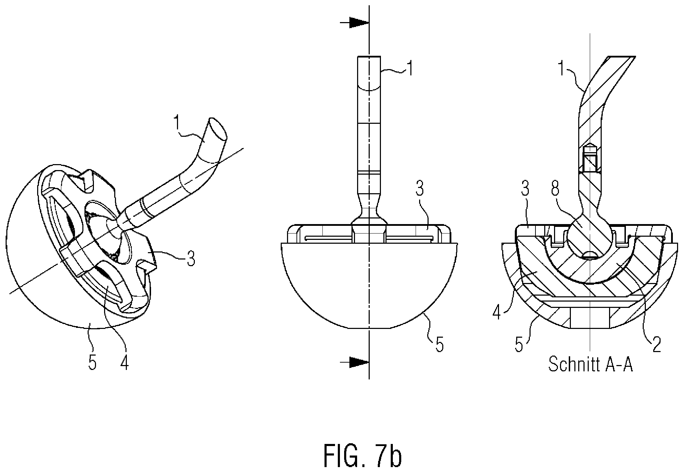

Figur 7a zeigt das Impaktierinstrument 1 montiert mit Einsetzhilfe 3, Impaktierkopf 2 und Pfanneneinsatz 4 vor Kontakt mit der Hüftpfanne 5 und Figur 7 b nach Aufsetzen auf die Hüftpfanne 5.

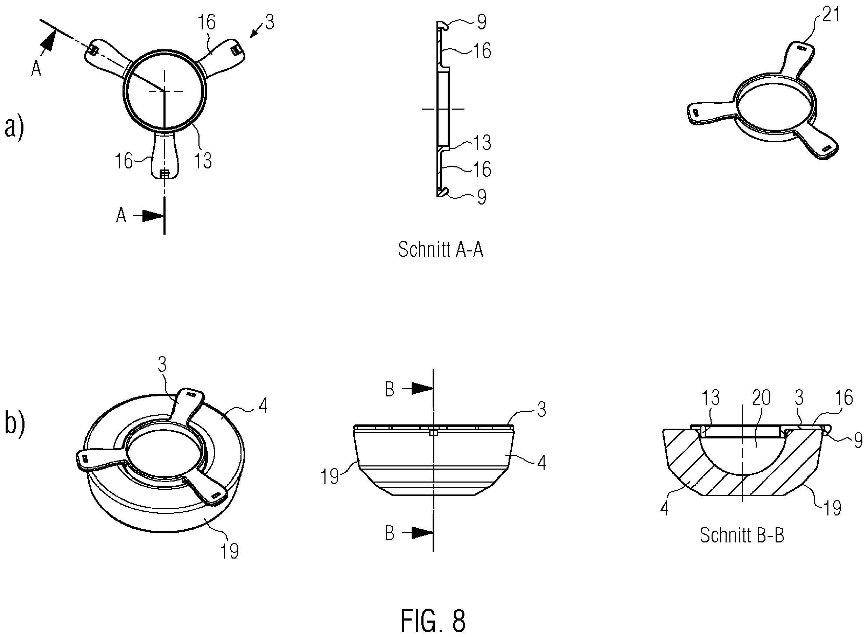

Figur 8 a zeigt eine Einsetzhilfe 3 mit 3 Halteklauen als separates Bauteil und Figur 8b aufgesetzt und verankert auf dem Pfanneneinsatz 4. Figur 8 c zeigt einen Schnitt von Figur 8 b.

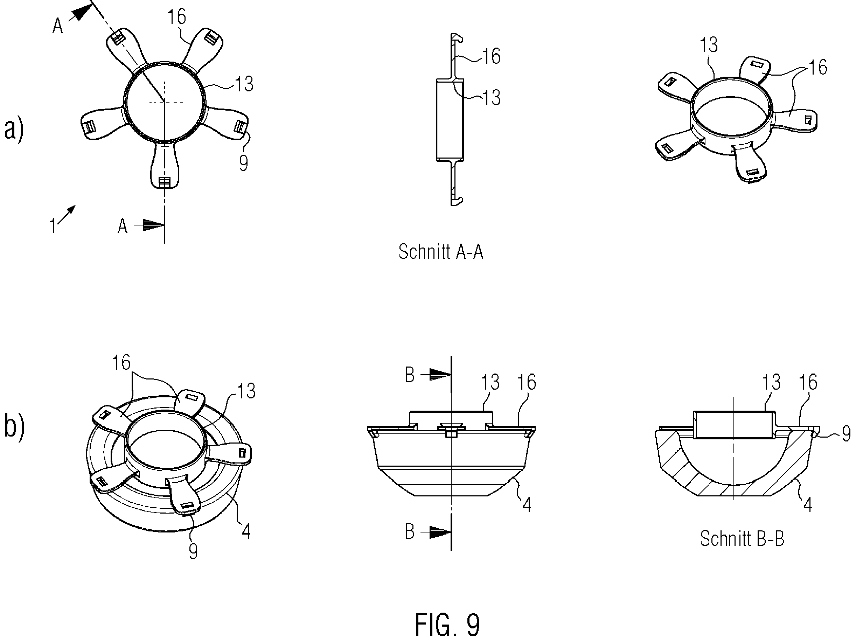

Figur 9 a zeigt eine Einsetzhilfe 3 mit 5 Halteklauen als separates Bauteil und Figur 9 b aufgesetzt und verankert auf dem Pfanneneinsatz 4. Figur 9 c zeigt einen Schnitt von Figur 9 b.

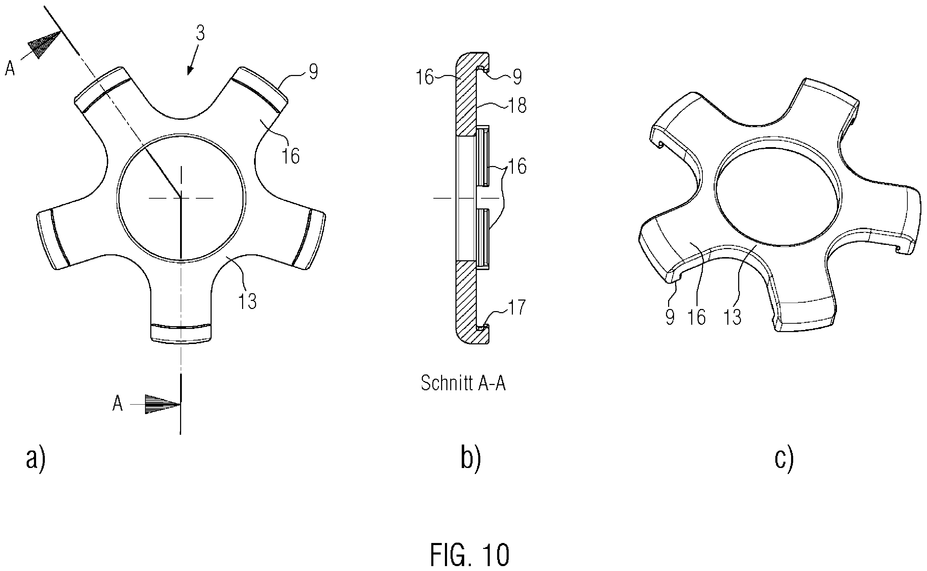

Figur 10 a zeigt eine alternative Einsetzhilfe 3 auch mit 5 Halteklauen als separates Bauteil in einer Draufsicht, Figur 10 a, im Schnitt, Figur 10 b und in Ansicht, Figur 10 c.

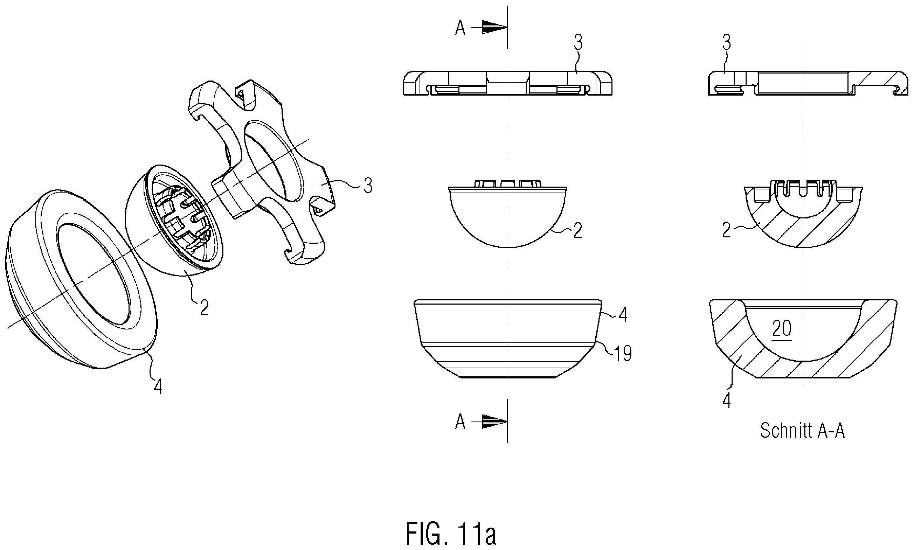

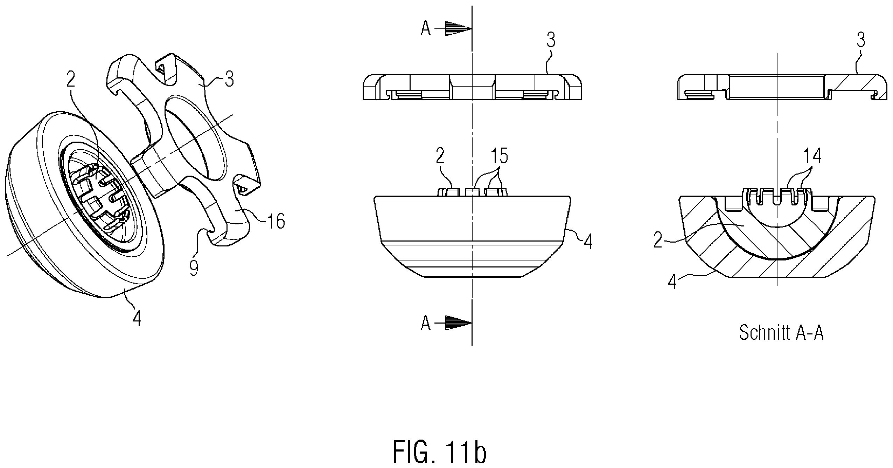

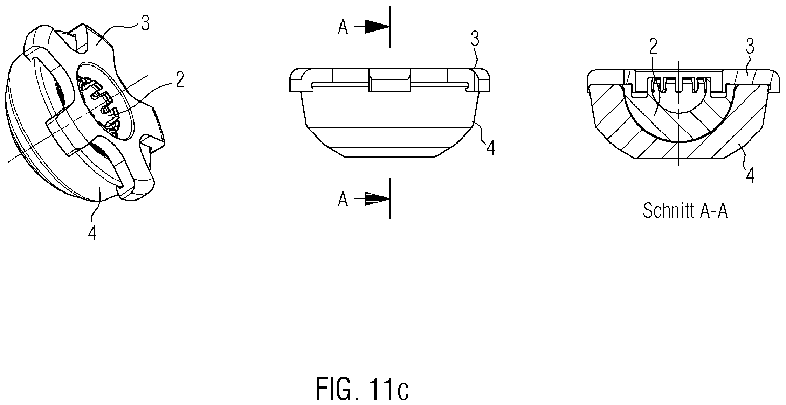

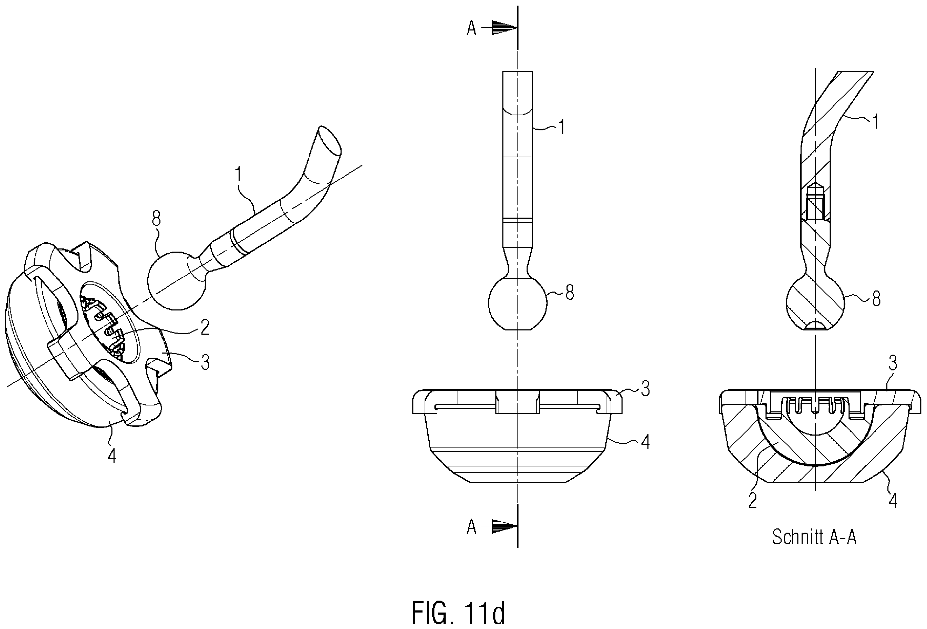

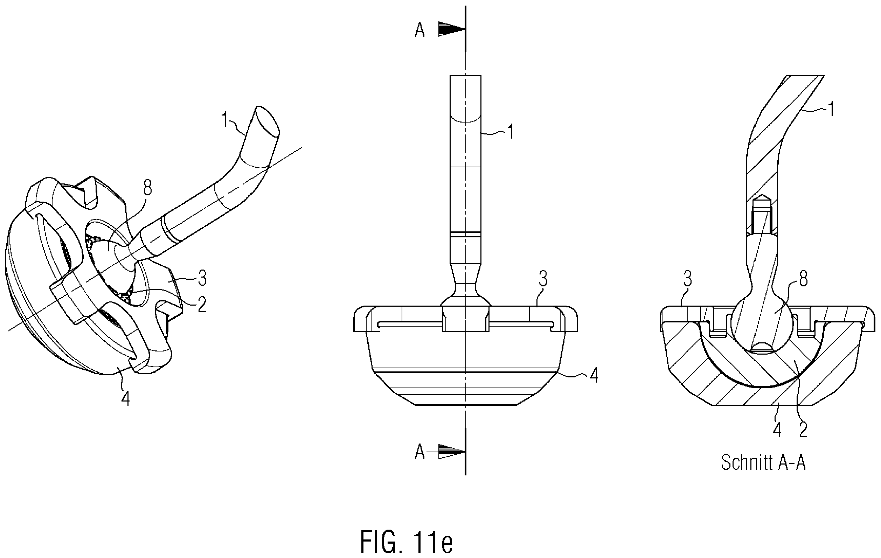

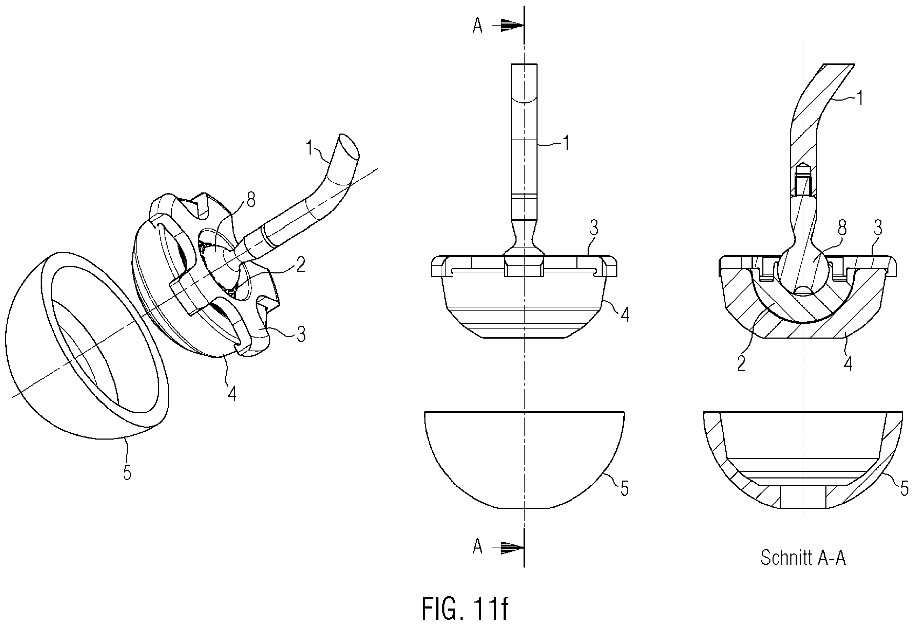

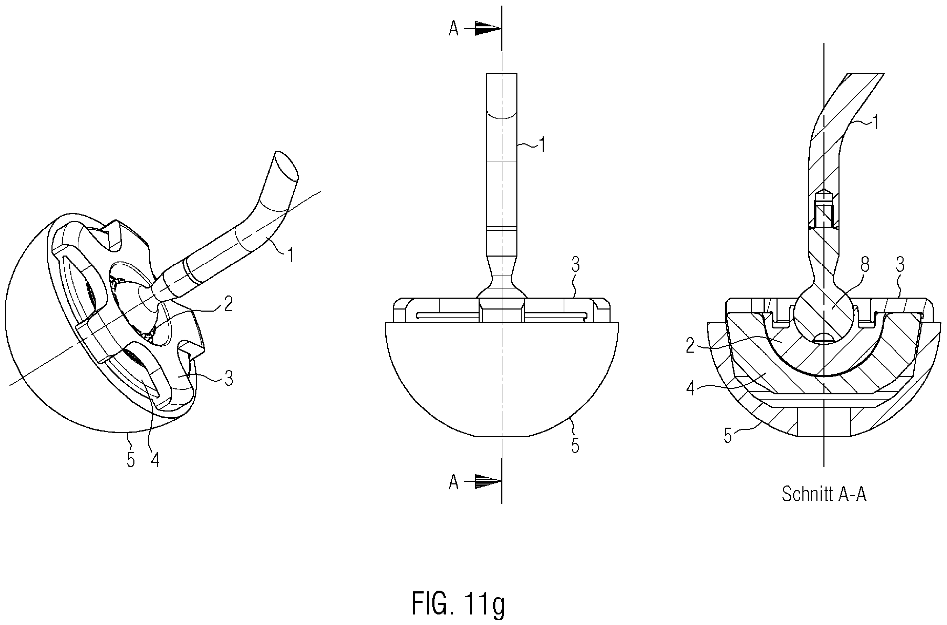

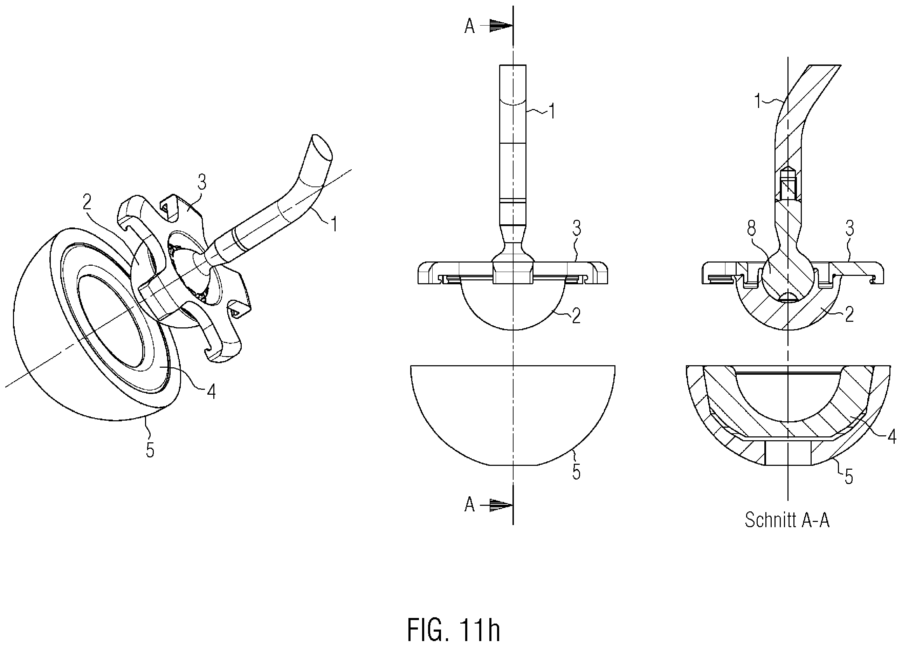

Die Figuren 11 a-h zeigen das erfindungsgemäße Verfahren im Verlauf des Zusammenbaus. Figur 11 a zeigt die Einsetzhilfe 3, den Impaktierkopf 2 und den Pfanneneinsatz 4 als Einzelteile vor dem Fügen.
   Figur 11 b zeigt den in den Pfanneneinsatz 4 eingelegten Impaktierkopf 2.
   Figur 11 c zeigt die Einsetzhilfe 3, die über den Pfanneneinsatz 4 mit eingelegtem Impaktierkopf 2 gesetzt wird und deren Halteklauen über den Rand des Pfanneneinsatzes 4 gezogen werden.
   Figur 11 d zeigt die zusammengefügten Bauteile Einsetzhilfe 3, den Impaktierkopf 2 und den Pfanneneinsatz 4 vor dem Fügen mit dem Impaktierinstrument 1.
   Figur 11 e zeigt den montierten Zustand der Schnappverbindung zwischen Impaktierinstrument 1 und Impaktierkopf 2.
   Figur 11 f zeigt den Zustand des komplett montierten Sets beim Einführen in das OP Feld vor Kontakt mit der Hüftpfanne 5.
   Figur 11 g zeigt den eingeführten Pfanneneinsatz 4 mit Einsetzhilfe 3, die mit ihrer Unterseite an der Stirnseite der Hüftpfanne 5 aufliegt.
   Figur 11 h zeigt den in der Hüftpfanne 5 verankerten Pfanneneinsatz 4 und die nach dem Ablösen vom Pfanneneinsatz noch verbundenen Einzelteile Impaktierkopf 2, Impaktierinstrument 1 und Einbringhilfe 3.

Das erfindungsgemäße Einsetzinstrument gemäß der Figuren besteht aus einem Impaktierinstrument 1, einem Impaktierkopf 2 und einer Einsetzhilfe 3 und verfügt über folgende funktionale Elemente bzw. Einzelteile:
- Impaktierinstrument 1 mit Haltegriff 6, auf dessen Ende beim Impaktieren ein Hammerschlag geführt wird
- gerader oder für minimal-invasive Techniken gekröpfter Stiel 7 dessen dem Haltegriff 6 abgewandtes Ende als Kugel 8 ausläuft.
- Optional kann nahe des kugelförmigen Endes, bzw. der Kugel 8 noch eine Auskragung 11 vorgesehen werden, die bei der Verkippung des angekoppelten Pfanneneinsatzes 4 als mechanischer Anschlag zur Bewegungsbegrenzung wirkt.
- kugelsegmentförmiger Impaktierkopf 2, der in etwa den Kugelkopfdurchmesser passend zur Kalotte des Pfanneneinsatzes 4 darstellt. Weiterhin verfügt der Impaktierkopf 2 über eine Kugelkalotte 14 mit nachgiebigem Kalottenrand 15, in die das kugelförmige Ende bzw. die Kugel 8 des Stieles 7 bei Montage einschnappt und so ein reibungsarmes Kugelgelenk bildet. Weiterhin verfügt der Impaktierkopf 2 über eine umlaufende Nut 12 zur Aufnahme des nachgiebigen Federringes 13 einer Einsetzhilfe 3 gem. DE 10 2009 054633 - siehe z. B. Figur 5.

Das hier beschriebene Einsetzinstrument ist als Erweiterung bzw. zusätzliche Instrumentierung zu einem Einsetzinstrument gemäß DE 10 2009 054633 (im weiteren "Einsetzhilfe" genannt) vorgesehen und wird bevorzugt in Kombination mit diesem verwendet. Es ermöglicht Operateuren, die einem direkten manuellen Einsetzen von Pfanneneinsätzen kritisch gegenüberstehen, das Handhaben und Fügen der Pfanneneinsätze mittels eines "klassischen" Instrumentes mit langem Stiel 7 und endseitigem Griff 6. Die Einsetzhilfe gemäß DE 10 2009 054633 wird dazu bevorzugt als Einwegprodukt bereitgestellt. Das hier beschriebene Einsetzinstrument, aus wenigen Einzelteilen bestehend, wird bevorzugt als wiederholt sterilisierbares Instrumentarium im sogenannten Sieb bereitgestellt.

Die modulare Kombination aus Einsetzhilfe als Einwegprodukt und Einsetzinstrument als Mehrwegprodukt bietet eine Reihe von Vorteilen im Vergleich zur Integration aller Funktionen in einem wiederverwendbaren Einsetzinstrument. Das Größenportfolio an Pfanneneinsätzen orientiert sich am Gleitpaarungsdurchmesser und am Außendurchmesser des Pfanneneinsatzes 4. Dabei ist es marktüblich, dass Pfanneneinsätze 4 mit gleichem Gleitpaarungs-durchmesser aber unterschiedlichen Außendurchmessern existieren. Der Außendurchmesser richtet sich dabei in der Regel nach dem Durchmesser der Hüftpfanne 5, in welche der Pfanneneinsatz 4 eingesetzt werden soll. Weiterhin ist es marktüblich zu einem Außendurchmesser Pfanneneinsätze 4 mit verschiedenen Gleitpaarungsdurchmessern anzubieten.

Um all diese unterschiedlichen Pfanneneinsätze 4 mit einem wiederverwendbaren Einsetzinstrument handhaben zu können, wäre es notwendig, bei *n* Gleitpaarungsdurchmessern und m möglichen Außendurchmessern genau *n x m* Impaktorköpfe 2 im Instrumentenset zur Verfügung zu stellen. Die hier beschriebene modulare Lösung eines zusätzlichen Einsetzinstrumentes zur vorhandenen Einsetzhilfe kommt hingegen mit *n* unterschiedlichen Impaktierköpfen 2 bei m unterschiedlichen Einsetzhilfen 3 aus.

Die intra-operative Montage der Bauteile erfolgt so, dass zuerst ein zum Gleitpaarungsdurchmesser des Pfanneneinsatzes 4 passender Impaktierkopf 2 in die Kalotte des Pfanneinsatzes 4 gesteckt wird. Anschließend wird eine zum Außendurchmesser des Pfanneneinsatzes 4 passende Einsetzhilfe 3 über den Pfanneneinsatz 4 mit eingelegtem Impaktierkopf 2 gesetzt und die Halteklauen der Einsetzhilfe 3 über den Rand des Pfanneneinsatzes 4 gezogen, so dass der Pfanneneinsatz 4 sicher von den Halteklauen gehalten wird - siehe Figur 11 a bis 11 c. Dann wird der im Pfanneneinsatz 4 gelagerte Impaktierkopf 2 auf das kugelförmige Ende des Impaktierinstruments 1 gesteckt und die Schnappverbindung Kugel-Kalotte gefügt. Der Pfanneneinsatz 4 ist damit fertig zur Handhabung und zum instrumentierten intra-operativen Einsetzen in die Hüftpfanne 5. Siehe Hierzu die Figuren 11 d-e.

Das Einsetzen beginnt mit dem Einführen des Pfanneneinsatzes 4 in das OP-Feld. Dabei ermöglicht das Kugelgelenk am Einsetzinstrument ein Ankippen des Pfanneneinsatzes 4 in Richtung der Achse des Stiels 7, um bei minimal-invasivem Zugang den Pfanneneinsatz 4 besser "einfädeln" zu können. Genauso erlaubt das Kugelgelenk ein seitliches "Anschlagen" bzw. "Hängenbleiben" des Pfanneneinsatzes 4 oder der Halteklauen 16 am Wundgewebe, ohne ein Abrutschen der Halteklauen 16 vom Pfanneneinsatz 4 zu riskieren. In dem Fall verkippt der Pfanneinsatz 4 im Kugelgelenk und gleitet unbeeinträchtigt weiter ins OP-Feld hinein.

Schließlich wird der Pfanneneinsatz 4 in die Hüftpfanne 5 hineinbewegt, bis die Halteklauen 16 an ihrer Unterseite die Stirnfläche der Hüftpfanne 5 berühren. Dadurch ist der Pfanneneinsatz 4 achsparallel zur Hüftpfanne 5 ausgerichtet. Auch dies läßt sich aufgrund des Kugelgelenkes am Einsetzinstrument einfacher erreichen, als bei bisherigen Lösungen. Siehe hierzu Figuren 11 f-g.

Sind Pfanneneinsatz 4 und Hüftpfanne 5 achsparallel ausgerichtet, kann der Pfanneneinsatz 4 impaktiert werden - siehe Figur 11 h. Zu diesem Zweck wird z. B. mittels eines marktüblichen OP-Hammers ein Impuls auf das freie Ende des Haltegriffs 6 ausgeführt. Der Impuls wird durch den Stiel 7 auf den Impaktierkopf 2 weitergeleitet. Der Impaktierkopf 2 bewegt schlagartig den Pfanneneinsatz 4 in Richtung Hüftpfanne 5. Dadurch wird der Pfanneneinsatz 4 aus den Halteklauen 16 und in weiterhin ausgerichteter Lage in die Hüftpfanne 5 geschoben, bis eine Verankerung der beiden Komponenten, z.B. durch konische Klemmung 10 auftritt. Die Einsetzhilfe 3 springt nach dem Auslösen der Halteklauen 16 ein Stück am Stiel 7 empor, bleibt aber weiterhin sicher am Stiel 7 hängen.

Je nach Empfehlung des Prothesenherstellers können anschließend weitere Schläge zur sicheren Verankerung der Komponenten ausgeführt werden. Anschließend wird das Einsetzinstrument aus dem OP-Feld entnommen. Aufgrund der konstruktiven Ausführung des Impaktierkopfes 2 ist gewährleistet, dass die Einsetzhilfe 3 nicht zufällig im OP-Feld verbleiben kann. So ist der radiale Durchmesser des Impaktierkopfes 2 größer als der Durchmesser des Federringes 13 der Einsetzhilfe 3. Dadurch wird die Einsetzhilfe 3 zwingend aus dem OP-Feld entnommen.

Nachfolgend werden bevorzugte erfinderische Ausgestaltungen der Einsetzhilfe 3 beschrieben, wie Sie einzeln oder in Verbindung mit dem oben beschriebenen Impaktorinstrument 1 und Impaktierkopf 2 verwendet werden können.

In den Figuren 8, 9 und 10 werden drei erfindungsgemäße Einsetzhilfen 3 bestehend aus einem ringförmigen, nachgiebigen Federring 13 mit Halteklauen 16 gezeigt.

Die Einsetzhilfe 3 (auch Einsetzinstrument genannt) gemäß Figur 8 besteht in dieser Ausführungsform aus einem nachgiebigen, ringförmigen Federring 13, an welchem sich drei Halteklauen 16 anschließen. Die Gestaltung der Einsetzhilfe 3 erfolgt dabei in fertigungstechnischer Hinsicht vorteilhaft als monolithisches Bauteil, welches z.B. durch Spritzgießen hergestellt werden kann. Die Nachgiebigkeit der Einsetzhilfe 3 führt dazu, dass die Halteklauen 16 radial verschoben werden können. Mit zunehmender radialer Verschiebung der Halteklauen 16 steigt dabei die zu überwindende Federkraft. Die Kraft-Weg-Kennlinie der Halteklauen 16 kann durch entsprechende geometrische Gestaltung des Federringes 13 beeinflusst werden.

Die Halteklauen 16 verfügen je über eine Haltefläche 17 und einer Auflagefläche 18 (siehe Figur 10 b). Die Halteklauen 16 liegen mit ihrer Auflagefläche 18 im montierten Zustand auf der Stirnfläche des Pfanneneinsatzes 4 auf. Die Halteflächen 17 liegen im montierten Zustand an der Außenfläche 19 des Pfanneneinsatzes 4 an. Im montierten Zustand greifen die Halteklauen 16 somit über die Stirnfläche und den äußeren Rand des Pfanneneinsatzes 4. Bevorzugt liegen der Federring 13 und die Halteklauen 16 in einer Ebene oberhalb der Kalotte 20 des Pfanneneinsatzes 4. Hierdurch lässt sich die Einsetzhilfe 3 leichter anfassen.

Figur 9 zeigt eine alternative erfindungsgemäße Einsetzhilfe 3 mit fünf Halteklauen 16, die im gleichen Abstand radial vom Federring 13 ausgehen.

Erfindungsgemäß ist die Einsetzhilfe 3 so dimensioniert, dass die Halteklauen 16 nur durch ein radiales Aufspannen über den äußeren Rand des Pfanneneinsatzes 4 geschoben werden können. Das heißt, der Federring 13 wird deformiert und auf die Halteklauen 16 wirkt eine Federkraft. Somit übt jede Haltefläche 17 (siehe Figur 9c) eine Druckkraft auf den Pfanneneinsatz 4 aus, welche der Federkraft an der jeweiligen Halteklaue 16 in etwa betrags- und richtungsgleich ist. Durch die Druckkräfte wirken zwischen den Halteflächen 17 und der Außenfläche 19 des Pfanneneinsatzes 4 ebenfalls Reibkräfte, die einem Abziehen der Einsetzhilfe 3 vom Pfanneneinsatz 4 entgegenwirken. Dies ist wesentlich für die Funktion der Einsetzhilfe 3.

Die Figur 9 b zeigt den Pfanneneinsatz 4 mit erfindungsgemäßer Einsetzhilfe 3 im montierten Zustand.

Wird der Pfanneneinsatz 4 mit montierter Einsetzhilfe 3 in eine Hüftpfanne 5 geschoben, kommen die Halteklauen 16 an ihrem unteren Ende in Kontakt mit der Stirnfläche der Hüftpfanne 5. Da die Halteklauen 16 alle die gleiche Ausdehnung nach unten haben, bilden die Kontaktpunkte eine Ebene, welche sowohl zur Stirnfläche der Hüftpfanne 5 als auch zur Stirnfläche des Pfanneneinsatzes 4 parallel ist. Somit erfolgt dadurch gleichfalls eine Ausrichtung der beiden Stirnflächen, so dass diese parallel zueinander liegen. Einer möglichen Verkippung des Pfanneneinsatzes 4 wird dadurch entgegengewirkt. Durch den zu diesem Zeitpunkt noch bestehenden, seitlichen Spalt zwischen Pfanneneinsatz 4 und Hüftpfanne 5 ist eine geringfügige Verschiebung des Pfanneneinsatzes 4 in der Hüftpfanne 5 möglich. Durch mehrfaches Hin- und Herschieben des Pfanneneinsatzes 4 in der Hüftpfanne 5 ist es dem Anwender möglich, die korrekte Position des Pfanneneinsatzes 4 in der Hüftpfanne 5 zu überprüfen. Gerade das Vorliegen leichter Verschiebbarkeit sowie das Anschlagen der Komponenten liefert dem Anwender eine sehr gutes palpatorisches Feedback über die korrekte Position des Pfanneneinsatzes 4 in der Hüftpfanne 5. Dies ist der Fall, wenn kein Impaktierinstrument 1 verwendet wird und der Anwender mit seinem Finger das Impaktierinstrument 1 ersetzt.

Um den Pfanneneinsatz 4 letztendlich bis zum reibschlüssigen Kontakt der beiden Konusflächen am Pfanneneinsatz 4 außen und an der Hüftpfanne 5 innen in die Hüftpfanne 5 hinein zu schieben, müssen die Reibkräfte zwischen den Halteflächen 17 der Einsetzhilfe 3 und der Außenfläche 19 des Pfanneneinsatzes 4 überwunden werden. Dies erfolgt durch kontinuierliche oder bevorzugt durch eine ruckartige Steigerung der axialen Fügekraft durch den Finger des Anwenders. Der Pfanneneinsatz 4 gleitet die verbliebene kurze Strecke in die Hüftpfanne 5 hinein, wobei keine nennenswerte Verkippung des Pfanneneinsatzes 4 mehr möglich ist. Mögliche geringfügige Verkippungen werden durch die selbstzentrierende Wirkung der konischen Klemmverbindung kompensiert und korrigiert.

An den äußeren Enden der Halteklauen 16 sind bevorzugt Durchstiche 21 zur leichteren Fertigung im Spritzgussverfahren angeordnet.

Die Ausführungsform der Einsetzhilfe 3 mit fünf anstatt nur drei Halteklauen 16 hat den Vorteil einer deutllich höheren Klemmung des Pfanneneinsatz 4. Zusätzlich ist im ungünstigen Falle beim Ablösen oder Abbrechen einer Halteklaue 16 während der Anwendung die Funktion des Einsetzens in die Hüftpfanne 5 gewährleitet.

Der im montierten Zustand der Einsetzhilfe 3 auf dem Pfanneneinsatz 4 nach oben über die Stirnfläche des Pfanneneinsatzes 4 hinausragende Federring 13 (siehe Figur 9) erlaubt dem Anwender ein besseres Handling / Gefühl beim Greifen im zusammengesetzen Zustand. Die axiale Höhe h des Federringes 13 (siehe Figur 9) liegt in der Ausführungsform gemäß Figuren 9 bevorzugt zwischen 6 und 12 mm, besonders bevorzugt zwischen 8 und 10 mm und in spezieller Ausführungsform bei 9 mm.

Die genannte Höhe h des Federrings 13 erlaubt neben der Standardauslösung über das Durchstecken eines Fingers und gleichzeitiges axiales Drücken auf den Kalottenboden des Pfanneneinsatzes 4 eine zusätzliche Möglichkkeit der Auslösung. Durch Greifen des Federrings 13 und einfaches Drücken auf den Federring 13 von oben löst sich ebenfalls die Halteverbindung und der Pfanneneinsatzes 4 wird in die Hüftpfanne 5 gedrückt und dort mittels konischer Klemmung positioniert und verankert.

Wie weiter oben beschrieben ist, kann anstatt der Finger das Impaktierinstrument 1 mit Impaktierkopf 2 verwendet werden. Beide Anwendungsarten (Finger oder Impaktierinstrument 1) führen zum gleichen Ergebnis, nämlich einer dauerhaften stabilen Verankerung des Pfanneneinsatzes 4 in der Hüftpfanne 5, wie in Figur 1 gezeigt. Ein verkippt eingesetzter Pfanneneinsatzes 4, wie in Figur 2 gezeigt, ist ausgeschlossen.

Figur 10 zeigt, wie schon erwähnt, eine weitere erfindungsgemäße Einsetzhilfe 3 mit fünf Halteklauen 16, die im gleichen Abstand radial vom Federring 13 ausgehen.

Die Einsetzhilfe 3 ist in dieser Ausführungsform so dimensioniert, dass die Halteklauen 16 nur durch ein radiales Aufspannen über den äußeren Rand des Pfanneneinsatzes 4 geschoben werden können. Das heißt, der Federring 13 wird deformiert und auf die Halteklauen 16 wirkt eine Federkraft. Somit übt jede Halteklauen 16 über ihre Haltefläche 17 (siehe Figur 10 c) eine Druckkraft auf den Pfanneneinsatz 4 aus, welche der Federkraft an der jeweiligen Halteklaue 16 betrags- und richtungsgleich ist. Dies ist wesentlich für die Funktion der Einsetzhilfe 3.

Die Form der Einsetzhilfe ist dabei mit höherem Materialeinsatz über das komplette Bauteil hinweg ausgeführt.

Der Übergang zwischen Federing 13 und Halteklauen 16 ist fließend geschwungen ausgeführt. Federing 13 und Halteklauen 16 haben bevorzugt die gleiche axiale Höhe, diese liegt im Bereich zwischen 3 und 5 mm.

Die Halteklauen 16 mit den Halteflächen 17 sind in der Breite durchgehend identisch ausgeführt. Diese liegt bevorzugt zwischen 10 und 14 mm, in spezieller Ausführungsform bei 12 mm. Die direkten Halteflächen 17 sind bevorzugt radial an die Aussenfläche 19 des Pfanneinsatzes 4 angepasst. Über die damit verbundene größere Kontaktfläche werden die Reibkräfte erhöht.

Diese geometrische Gestaltung erhöht die Steifigkeit des Bauteils mit dem Vorteil einer deutlich höheren Klemmung des Pfanneneinsatzes. Der Widerstand gegen ein vorzeitiges Ablösen der Einsetzhilfe 3 vom Pfanneneinsatz 4 beim Kontakt mit der Umgebung wird dadurch erhöht.

Durch die hohe Klemmkraft bzw. zu überwindende Auslösungskraft bei der Anwendung passen Einsetzhilfe 3, Impaktierinstrument 1 und Impaktierkopf 2 exzellent zueinander.

Um den Pfanneneinsatz 4 durch das Überwinden der Reibkräfte zwischen den Halteflächen 17 der Einsetzhilfe 3 und der Außenfläche 19 des Pfanneneinsatzes 4 gleichmäßig zu positionieren wird eine ruckartige Steigerung der axialen Fügekraft über einen leichten Schlag mit der flachen Hand auf das Ende des Impaktierinstruments 1 empfohlen.

Die hier beschriebene Einsetzhilfe 3 wird bevorzugt als wiederholt sterilisierbares Produkt zusammen mit dem Impaktierinstrument 1 und Impaktierkopf 2 im so genannten Sieb bereitgestellt. Die Wahl des Werkstoffs der Einsetzhilfe entspricht den Anforderungen hinsichtlich der Beständigkeit bei wiederholten Aufbereitungszyklen (Dampfsterilisation bei 134°C).

## Patentansprüche

1. Einsetzinstrument für eine Hüftgelenkprothese zum instrumentierten Einsetzen mit einem Impaktierinstrument (1) mit einem Stiel (7), an dessen einem Ende (8) sich ein Haltewerkzeug befindet, wobei das eine Ende (8) des Impaktierinstruments (1) ein Teil des Haltewerkzeugs ist, **dadurch gekennzeichnet, dass** das Einsetzinstrument geeignet ist zum Einsetzen eines Pfanneneinsatzes (4) mit einer kugelförmigen Kalotte (20) in eine Hüftpfanne (5) der Hüftgelenkprothese, wobei das eine Ende (8) des Impaktierinstruments (1) kugelförmig ausgebildet ist und das Haltewerkzeug weiterhin einen als separates Bauteil ausgebildeten Impaktierkopf (2) zur gelenkigen Aufnahme des kugelförmigen Endes (8) des Impaktierinstruments (1) und eine als separates Bauteil ausgebildete Einsetzhilfe (3) zum Halten des Pfanneneinsatzes (4) auf der Außengeometrie des Impaktierkopfs (2) umfasst und der Impaktierkopf (2) mit dem kugelförmigen Ende (8) ein Kugelgelenk bildend verbunden ist und die Einsetzhilfe (3) aus einem Federring (13) mit radial abstehenden Halteklauen (16) besteht, wobei die Halteklauen (16) aufgrund der Nachgiebigkeit des Federrings (13) radial verschiebbar sind und am äußeren Ende der Halteklauen (16) axial abstehende Haken (9) angeordnet sind, die im montierten Zustand mit ihrer Haltefläche (17) an der Außenfläche (19) des Pfanneneinsatzes (4) anliegen und zugleich die Halteklauen (16) mit ihrer Auflagefläche (18) auf dem Rand des Pfanneneinsatzes (4) aufliegen.

2. Einsetzinstrument nach Anspruch 1, **dadurch gekennzeichnet, dass** die Außengeometrie des Impaktierkopfs (2) an die Geometrie der Kalotte (20) des Pfanneneinsatzes (4) angepasst ist.

3. Einsetzinstrument nach Anspruch 1 oder 2, **dadurch gekennzeichnet, dass** der Impaktierkopf (2) über eine Kugelkalotte (14) mit nachgiebigem Kalottenrand (15) verfügt, in die das kugelförmige Ende (8) des Impaktierinstruments (1) bei Montage einschnappt.

4. Einsetzinstrument nach Anspruch 3, **dadurch gekennzeichnet, dass** der Kalottenrand (15) koaxial von einer umlaufenden Nut (12) umgeben ist.

5. Einsetzinstrument nach einem der Ansprüche 1 bis 4, **dadurch gekennzeichnet, dass** der Federring (13) und die Halteklauen (16) im montierten Zustand in einer Ebene oberhalb der Kalotte (20) des Pfanneneinsatzes (4) liegen.

6. Einsetzinstrument nach einem der Ansprüche 1 bis 5, **dadurch gekennzeichnet, dass** oberhalb des kugelförmigen Endes (8) des Impaktierinstruments (1) eine Auskragung (11) zur Bewegungsbegrenzung des Kugelgelenks (8, 14) angeordnet ist.

7. Einsetzinstrument nach einem der Ansprüche 1 bis 6, **dadurch gekennzeichnet, dass** der radiale Durchmesser des Impaktierkopfes (2) größer als der Durchmesser des Federringes (13) der Einsetzhilfe (3) ist.

## Claims

1. Insertion instrument for a hip joint prothesis for instrumental insertion by means of an impacting instrument (1) having a shaft (7), at one end (8) of which a holding tool is located, the one end (8) of the impacting instrument (1) being part of the holding tool, **characterized in that** the insertion instrument is suitable for inserting a socket insert (4) comprising a spherical cap (20) into a hip socket (5) of the hip joint prothesis, the one end (8) of the impacting instrument (1) being spherical, and the holding tool further comprising an impacting head (2), which is designed as a separate component, for receiving the spherical end (8) of the impacting instrument (1) in an articulated manner, and an insertion aid (3), which is designed as a separate component, for holding the socket insert (4) on the external geometry of the impacting head (2), and the impacting head (2) being connected to the spherical end (8) so as to form a ball-and-socket joint, and the insertion aid (3) consisting of a spring lock washer (13) comprising radially projecting holding claws (16), the holding claws (16) being radially movable owing to the compliance of the spring lock washer (13), and axially projecting hooks (9) being arranged on the outer end of the holding claws (16) and, when mounted, abutting the outer surface (19) of the socket insert (4) by means of their holding surfaces (17) and at the same time the holding claws (16) resting on the rim of the socket insert (4) by means of their bearing surfaces (18).

2. Insertion instrument according to claim 1, **characterized in that** the external geometry of the impacting head (2) is adapted to the geometry of the cap (20) of the socket insert (4).

3. Insertion instrument according to either claim 1 or claim 2, **characterized in that** the impacting head (2) comprises a spherical cap (14) which has a compliant cap edge (15) and into which the spherical end (8) of the impacting instrument (1) snaps during assembly.

4. Insertion instrument according to claim 3, **characterized in that** the cap edge (15) is surrounded coaxially by a circumferential groove (12).

5. Insertion instrument according to any of claims 1 to 4, **characterized in that** the spring lock washer (13) and the holding claws (16) lie in a plane above the cap (20) of the socket insert (4) when mounted.

6. Insertion instrument according to any of claims 1 to 5, **characterized in that** an overhang (11) for limiting the movement of the ball-and-socket joint (8, 14) is arranged above the spherical end (8) of the impacting instrument (1).

7. Insertion instrument according to any of claims 1 to 6, **characterized in that** the radial diameter of the impacting head (2) is larger than the diameter of the spring lock washer (13) of the insertion aid (3).

## Revendications

1. Instrument d'insertion pour une prothèse de hanche destiné à la mise en place assistée avec un instrument d'impaction (1) avec une tige (7) à une extrémité (8) de laquelle se trouve un outil de retenue, l'extrémité (8) de l'instrument d'impaction (1) étant une partie de l'outil de retenue, **caractérisé en ce que** l'instrument d'insertion est approprié pour la mise en place d'un insert acétabulaire (4) avec une calotte (20) de forme sphérique dans une cupule acétabulaire (5) de la prothèse de hanche, l'extrémité (8) de l'instrument d'impaction (1) étant constituée sous forme sphérique, et l'outil de retenue comprenant en outre une tête d'impaction (2), constituée en tant que composant séparé, pour la réception articulée de l'extrémité (8) de forme sphérique de l'instrument d'impaction (1), et un auxiliaire d'insertion (3), constitué en tant que composant séparé, pour la retenue de l'insert acétabulaire (4) sur la géométrie extérieure de la tête d'impaction (2), et la tête d'impaction (2) étant raccordée à l'extrémité (8) de forme sphérique en formant une articulation sphérique, et l'auxiliaire d'insertion (3) se composant d'une bague élastique (13) avec des griffes de retenue (16) dépassant radialement, les griffes de retenue (16) pouvant être déplacées radialement en raison de la flexibilité de la bague élastique (13), et des crochets (9) dépassant axialement étant disposés à l'extrémité extérieure des griffes de retenue (16) et étant, dans l'état monté, adjacents à la surface extérieure (19) de l'insert acétabulaire (4) par leur surface de retenue (17), et les griffes de retenue (16) reposant dans le même temps sur le bord de l'insert acétabulaire (4) par leur surface d'appui (18).

2. Instrument d'insertion selon la revendication 1, **caractérisé en ce que** la géométrie extérieure de la tête d'impaction (2) est adaptée à la géométrie de la calotte (20) de l'insert acétabulaire (4).

3. Instrument d'insertion selon la revendication 1 ou 2, **caractérisé en ce que** la tête d'impaction (2) dispose d'une calotte sphérique (14), avec un bord de calotte (15) flexible, dans laquelle l'extrémité (8) de forme sphérique de l'instrument d'impaction (1) s'enclenche lors du montage.

4. Instrument d'insertion selon la revendication 3, **caractérisé en ce que** le bord de calotte (15) est entouré de façon coaxiale d'une rainure (12) périphérique.

5. Instrument d'insertion selon l'une des revendications 1 à 4, **caractérisé en ce que**, dans l'état monté, la bague élastique (13) et les griffes de retenue (16) sont situées dans un plan au-dessus de la calotte (20) de l'insert acétabulaire (4).

6. Instrument d'insertion selon l'une des revendications 1 à 5, **caractérisé en ce que**, au-dessus de l'extrémité (8) de forme sphérique de l'instrument d'impaction (1), il est disposé une protubérance (11) destinée à limiter le mouvement de l'articulation sphérique (8, 14).

7. Instrument d'insertion selon l'une des revendications 1 à 6, **caractérisé en ce que** le diamètre radial de la tête d'impaction (2) est supérieur au diamètre de la bague élastique (13) de l'auxiliaire d'insertion (3).
